# DEMANDE DE BREVET EUROPEEN

(11) **EP 3 698 825 A1**
(43) Date de publication de la demande: **26.08.2020**
(21) Numéro de dépôt: 20159341.5
(22) Date de dépôt: 16.10.2015
(51) Int. Cl.: A61M 1/28

(54) **SYSTÈME D'ADMINISTRATION ET MODE DE FONCTIONNEMENT CORRESPONDANT**

(30) Priorité: 17.10.2014 EP 14189455
(62) Demande divisionnaire de: 15801237.7
(71) Demandeur: Debiotech S.A., 1004 Lausanne (CH)
(72) Inventeur: NEFTEL, Frédéric, 1004 Lausanne (CH); THIEBAUD, Pierre, 2088 Cressier (CH)
(74) Mandataire: Weihs, Bruno Konrad

(57) **Abrégé**

Système médical adapté pour délivrer un fluide à un patient selon plusieurs modes de fonctionnement dont un mode sécurisé qui permet en outre de continuer la délivrance ou le traitement en cas de détection d'une probable anomalie.

## Description

### Domaine de l'invention

L'invention concerne un système médical adapté pour délivrer un fluide à un patient selon plusieurs modes de fonctionnement dont un mode sécurisé. Ledit mode sécurisé permet en outre de continuer la délivrance ou le traitement en cas de détection d'une probable anomalie. La présente demande revendique la priorité de la demande portant le numéro EP 14189455.0, déposée le 17 octobre 2014 au nom de Debiotech, dont le contenu intégral doit être considéré comme faisant partie de la présente demande.

### Etat de la technique

Le dispositif présentement divulgué peut être adapté à de nombreux dispositif de délivrance. Il est toutefois particulièrement adapté pour les traitements par dialyse péritonéale.

La dialyse péritonéale est un moyen thérapeutique permettant de purifier le sang. Elle permet, à un patient souffrant d'une insuffisance rénal, d'éliminer les impuretés telles que l'urée et l'excès d'eau de l'organisme qui auraient été habituellement éliminés par des reins fonctionnant normalement. Ce moyen thérapeutique utilise le péritoine du patient. La membrane péritonéale dispose d'une très grande surface et elle comporte de très nombreux vaisseaux sanguins. Elle joue ainsi le rôle de filtre naturel entre le sang et tout liquide éventuellement présent dans la cavité péritonéale. De nombreux brevets divulguent des systèmes permettant d'effectuer des dialyses péritonéales (EP 1 648 536 A2, EP 0 471 000 B1, EP 1 195 171 B1, EP 1 648 536 B1 qui sont intégrés par référence à la présente description) pour l'injection et le retrait du fluide dans le péritoine du patient.

Le traitement par dialyse péritonéale est relativement simple et comprend au moins un cycle de trois phases distinctes :
- le « Fill » : le système injecte du dialysat dans la cavité péritonéale du patient (appelé également phase d'injection) ;
- le « Dwell » : le système laisse durant un temps déterminé le dialysat dans la cavité péritonéale (appelé également phase de stase) ;
- le « Drain » : le système retire le dialysat présent dans la cavité péritonéale (appelé également phase de drainage).
Dans le présent document, une phase peut être un Fill, un Dwell ou un Drain (chaque phase pouvant être totale ou partielle), un cycle comprend un Fill, un Dwell et un Drain et le traitement peut comprendre plusieurs cycles. Autrement dit, les phases peuvent être répétées durant un même traitement.

Les systèmes généralement appelés APD (Automated Peritoneal Dialysis) sont adaptés pour effectuer plusieurs phases de Fill, de Dwell et de Drain qui se succèdent, autrement dit plusieurs cycles se succédant durant un même traitement. Ce type de système effectue ainsi un traitement pendant plusieurs heures. Aussi, les APD sont particulièrement adaptées pour une utilisation durant la nuit et/ou chez le patient.

De tels systèmes comprennent des moyens adaptés pour contrôler et/ou surveiller leur bon fonctionnement. Ces moyens peuvent mesurer ou estimer ou calculer les volumes injecté(s) ou retiré(s) du péritoine. Par exemple, ces moyens peuvent comprendre un capteur connecté à un processeur. Le contrôle de ces volumes est d'une importance capitale. En effet, le système ne doit en aucun cas injecter une trop grande quantité de dialysat dans la cavité péritonéale, ni laisser un volume important à la fin du traitement ou à la fin de chaque phase de Drain. Cela pourrait avoir plusieurs conséquences sur la santé du patient (détérioration du péritoine, œdème pulmonaire, perte de la capacité d'ultra-filtration, insuffisance respiratoire ou cardiaque,...) et, pour le moins, altérer le confort du malade s'il n'entraine pas des conséquences vitales.

Dans le cas où un capteur est défectueux et que le système ne détecte pas cette défectuosité, le capteur peut causer une sur ou sous-estimattion des volumes injectés et/ou retirés durant le traitement. Cette erreur est d'autant plus importante lorsque le traitement comprend plusieurs cycles car dans ce cas, l'erreur d'estimation est répété et cumulée à chaque cycle. Cette erreur peut être la conséquence d'un ou plusieurs facteurs, tels que l'usure ou un défaut (temporaire ou non) de la machine, du système de pompage, des capteurs, le déplacement du patient, l'évolution de la température... Dans le cas où le système comprend une partie jetable (tube, cassette, réservoir,...) et une partie réutilisable (machine, électronique, capteur), il peut également s'agir d'une mauvaise liaison / accouplement entre les deux parties.

Lors d'une anomalie, les systèmes de l'art antérieur alertent simplement le patient ou le personnel médical afin qu'il puisse effectuer une ou plusieurs actions pour corriger le problème. Certains systèmes très prévoyants préfèrent même alerter l'utilisateur même lors de la détection d'une anomalie potentielle.

Dans le cas où le traitement s'effectue durant la nuit et/ou chez le patient, certaines alarmes peuvent déranger le sommeil du patient sans que cela soit réellement nécessaire et/ou alerter inutilement le patient alors que ce dernier n'a pas la capacité d'intervenir. De plus, la cause réelle du déclenchement de l'alarme peut parfois être le fait que le patient ait simplement bougé durant le traitement. Ainsi, il serait inutile de réveiller le patient comme le font les systèmes de l'art antérieur, car le défaut ne serait que temporaire et n'engagerait pas réellement la sécurité du patient.

Dans d'autres circonstances, l'anomalie peut persister ou du moins un doute sur l'anomalie peut conduire le système de l'art antérieur à arrêter prématurément le système, ce qui conduit à interrompre un traitement qui est nécessaire au patient. De façon générale, les systèmes de l'art antérieur favorisent l'interruption d'un traitement dès lors qu'un disfonctionnement pourrait conduire à un risque pour le patient. En particulier, aucun système de l'art antérieur ne prévoit une modification du traitement afin de limiter ce risque patient tout en continuant à opérer en la présence d'un tel disfonctionnement.

### Description générale de l'invention

L'invention présenté dans ce document apporte plus d'intelligence sur le traitement des données et/ou le fonctionnement du système afin d'optimiser le traitement même en cas d'une anomalie ou en cas de détection d'une potentiel anomalie. En particulier, l'invention peut commuter de mode de fonctionnement (c'est-à-dire, par exemple, modifier un ou plusieurs paramètres du traitement) après détection d'une possible anomalie, ce nouveau mode de fonctionnement peut être appelé « sécurisé» car il est éventuellement moins efficace que le mode d'origine, mais il permet d'apporter au patient un traitement qui reste plus favorable que l'arrêt prématuré du traitement tout en permettant tout de même de garantir la sécurité du patient. Le principe de l'invention est un système adapté pour garantir un traitement minimal (le plus favorable au patient en fonction des circonstances données, par exemple en fonction du niveau de connaissance de l'état du système et/ou de l'environnement du patient) tout en assurant un effet favorable pour la santé du patient.

En d'autres termes, le système inventé permet de réaliser un traitement moins efficace lorsqu'il détecte une défectuosité, qui obéit à des spécifications moins contraignantes (par exemple durée du traitement, débit du fluide,...) que celle d'un système ne comprenant pas de défaut mais qui néanmoins garantit la sûreté du patient et la continuité de son traitement. Ainsi, contrairement aux systèmes de l'art antérieur, dans le cas où un ou plusieurs défauts ou fautes seraient détectés, ou serait soupçonnés, au lieu de stopper le traitement et de se mettre en état d'alarme (et ainsi déranger le patient et ne lui délivrer qu'un traitement incomplet, insuffisant et potentiellement nocif), le système inventé ajustera le traitement (certains ou tous les paramètres qui le décrive) pour garantir qu'ilsoit mené à bout, que la précision requise pour la sécurité du patient sera maintenue, mais sans garantie que les spécifications de la machines soient respectées.

Le système présenté dans le document est particulièrement adapté pour les systèmes de dialyse péritonéale et encore plus pour les systèmes de Dialyse Péritonéale Automatisé (ADP) du fait de la répétition des cycles. En effet, l'addition de cycle répétitif peut avoir un impact très fort sur l'imprécision d'un dispositif en cas de défaut de celui-ci entrainant un risque de faute systématique. A chaque cycle celle-ci s'additionnera à la précédente pour générer, au bout de plusieurs cycle un effet très important.

Un premier aspect de l'invention est relatif à un dispositif médical qui comprend au moins deux modes de fonctionnement. Un premier mode de fonctionnement dit normal où le système définit un ensemble de paramètres (par exemple, un volume, un débit, le nombre de cycle,...) afin d'atteindre l'effet escompté par une certaine prescription thérapeutique. Un deuxième mode de fonctionnement dit « sécurisé » où le système a détecté une défaillance ou une anomalie ou un événement perturbateur qui contraint le système à modifier au moins un desdits paramètres afin de continuer le traitement selon un nouvel ensemble de paramètres (par exemple, diminuer ou augmenter le volume, le débit et/ou les cycles,...). Ledit mode de fonctionnement sécurisé ne permet éventuellement pas d'atteindre le même niveau d'efficacité (cela peut se mesurer à travers par exemple la quantité d'ultrafiltrat, la durée du traitement,...) que le mode de fonctionnement normal. Toutefois, ce mode de fonctionnement permet d'atteindre une efficacité minimum du traitement, notamment plus favorable que l'interruption du traitement, tout en garantissant la sécurité du patient.

Selon un deuxième aspect de l'invention, le dispositif comprend un mode de fonctionnement sécurisé, comme décrit précédemment, mais dont les paramètres peuvent s'adapter en fonction de l'importance de l'anomalie et/ou de son évolution. Autrement dit, le mode sécurisé est moins efficace que le mode normal, ce mode permettant de garantir la sécurité du patient tout en adaptant les paramètres afin de procurer au patient un traitement optimal en fonction des circonstances effectives. Par exemple, il peut s'agir de la détection d'une dérive d'un capteur et au fur et à mesure que les données du capteur dérivent, le dispositif adapte les paramètres tels qu'une baisse progressive du débit ou du volume de fluide injecté ou augmentation du volume drainé. En fonction des mesures réalisées sur les différents capteurs, et si le doute sur la présence d'un défaut augmente ou que l'estimation du risque d'impact de ce défaut sur la qualité du traitement ou sur le risque que pourrait courir le patient augmente, le mode de fonctionnement sécurisé va adapter les paramètres du traitement entrainant à chaque fois une baisse de l'efficacité du traitement mais garantissant que le nouveau traitement restera sûr pour le patient.

Dans un mode de réalisation, le système comprend au moins un capteur (capteur de pression, de température, de débit, ...) relié à un processeur destiné à définir l'état du système ou surveiller un paramètre du traitement ou de l'environnement du système. Le processeur peut être adapté pour reconnaitre une défaillance de ce capteur ou supposer une défaillance de ce capteur. Ceci peut se faire soit en dédoublant les capteurs, et comparer les valeurs données par les deux entités. Une différence signifie que l'un des éléments au moins est en défaut. Cela peut aussi se faire en comparant la réponse du capteur avec une courbe théorique idéale. En cas de différence trop prononcée, on considérera que le capteur est en défaut. Si la défaillance de ce capteur peut être négligée, le système peut décider de continuer le traitement dans un mode de fonctionnement normal. Si la défaillance de ce capteur engendre des conséquences pouvant potentiellement mettre en cause la sécurité du patient, alors le système à la capacité de définir un nouvel ensemble de paramètres selon un mode de fonctionnement sécurisé capable de poursuivre le traitement et de garantir la sécurité du patient même en présence de l'anomalie détectée ou suspectée.

Durant le traitement, l'anomalie peut évoluer, par exemple s'amplifier, et le système est alors (à nouveau) adapté pour redéfinir un nouvel ensemble de paramètres en conséquence qui permettront de garantir la sécurité du patient jusqu'à la fin du traitement, même si celui-ci perd de l'efficacité. Par exemple, si un capteur permettant de calculer, mesurer ou estimer les volumes injecté(s) ou drainé(s) est défaillant, le système peut diminuer la quantité injectée ou augmenter la quantité drainée afin de limiter les risques de sur-remplissage du patient pouvant résulter d'une telle défaillance de ce capteur. A chaque cycle et durant les différentes phases, le système peut graduellement modifier un ou plusieurs paramètres : volume déplacé, le débit de la pompe, la température,...

Dans un mode réalisation, le système comprend deux capteurs assurant la redondance de la mesure d'un paramètre assurant la sécurité du patient. Ces capteurs peuvent par exemple mesurer (ou calculer avec l'aide du processeur) le débit afin de s'assurer de la précision du débit du dialysat injecté ou drainé. En cas de défaillance d'au moins un des deux capteurs, le mode sécurisé sera de préférence activé assurant ainsi la sécurité du patient sur la base d'un seul capteur fonctionnel. La défaillance de l'un des deux capteurs peut être détecté du fait que l'écart entre les deux capteurs dépasse un certain seuil ou que les profils de pression ne sont pas cohérent l'un par rapport à l'autre ou par rapport à l'actionnement de la pompe.

Dans les paragraphes précédents la défaillance décrite est celle d'un capteur. Il est évident pour l'homme du métier, qu'il peut s'agir de n'importe quelle défaillance du système. Celle-ci peut toucher un capteur mais peut aussi bien toucher n'importe quelle partie du système et être identifiée par un capteur présent dans le système.

Selon un troisième aspect de l'invention, le dispositif à la capacité de décider de maintenir (en mode normal ou sécurisé) le traitement dans des conditions optimales pour la sécurité du patient ou d'arrêter prématurément le traitement en fonction de l'efficacité escomptée de chacune de ces options (continuer le mode de fonctionnement normal, commuter sur un mode de fonctionnement sécurisé, arrêt prématuré du traitement). Autrement dit, le système comprend un processeur adapté pour évaluer l'effet potentiel du traitement ou des traitements alternatifs et de comparer l'effet sur la santé du patient à celui résultant d'un arrêt prématurément du traitement. Ainsi, le système est capable de décider sans l'intervention du patient ou du personnel soignant, de la meilleure option pour la santé et la sécurité du patient en toutes circonstances. En particulier, on peut considérer que si le défaut est détecté à un stade avancé du traitement, par exemple lorsqu'un certain pourcentage, défini à l'avance, du traitement a été atteint, il est préférable d'arrêter le traitement au lieu de le poursuivre dans des conditions d'efficacité moindre.

Un quatrième aspect de l'invention est relatif à un procédé de commande d'un appareil de dialyse péritonéale comprenant les étapes suivantes :
- Observer au moins un paramètre relatif au traitement
- Déterminer une première plage de valeur acceptable pour ledit paramètre
- Commuter d'un mode de fonction à un premier mode de fonction sécurisé si les données dudit au moins paramètre sont en dehors de ladite première plage de valeur acceptable.
Le procédé peut comprendre une adaptation progressive de la plage de valeur acceptable et du mode de fonctionnement sécurisé adapté à cette plage de valeur pour ledit paramètre.

### Liste des figures

L'invention sera mieux comprise ci-après au moyen de quelques exemples illustrés. Il va de soi que l'invention ne se limite pas à ces modes de réalisation.
Figure 1 illustre le couplage entre une cassette et un cycler.
Figure 2 illustre plusieurs modes de fonctionnement possibles.
Figures 3 à 7 schématisent le fonctionnement possible d'un tel dispositif.
Figure 8 illustre brièvement un mode réalisation minimum.
Figures 9 à 11b schématisent le fonctionnement possible d'un tel dispositif.

### Références numériques utilisées dans les figures

- 1: cycler
- 2: cassette
- 3: entrée ou sortie de fluide
- 4: actionneur (valve)
- 5: capteur de pression
- 6: zone d'accouplement de la cassette avec un capteur de pression
- 7: mécanisme de pompage
- 8: actionneur (du mécanisme de pompage)
- 9: valve
- 10: capteur
- 11: processeur
- 12: mode de fonctionnement possible
- 20: paramétrage
- 21: activation de la pompe
- 22: première condition respectée?
- 23: commutation du mode de fonctionnement
- 24: précédents paramètres inchangés
- 25: deuxième condition respectée?
- 26: arrêt de la pompe
- 30: système de pompage
- 31: capteur de pression 1
- 32: capteur de pression 2
- 33: sens de l'écoulement du fluide propulsé par la pompe
- 34: processeur

### Description détaillée de l'invention

Dans le présent document, la description détaillée de l'invention comporte des modes de réalisation de dispositifs, de systèmes et de méthodes présentés à titre d'illustration. Il est bien entendu que d'autres modes de réalisation sont envisageables et peuvent être apportées sans s'écarter de la portée ou l'esprit de l'invention. La description détaillée qui suit, par conséquent, ne doit pas être prise dans un sens limitatif.

Sauf indication contraires, les termes scientifiques et techniques utilisé dans le présent document ont des significations couramment utilisés par l'homme du métier. Les définitions apportées dans ce document sont mentionnées afin de faciliter la compréhension des termes fréquemment utilisés et ne sont pas destinées à limiter la portée de l'invention.

Les indications de direction utilisées dans la description et les revendications, telles que "haut", "bas", "gauche", "droite", "supérieur", "inférieur", et autres directions ou orientations sont mentionnées afin d'apporter plus de clarté en référence aux figures. Ces indications ne sont pas destinées à limiter la portée de l'invention.

Les verbes « avoir », « comprendre », « inclure » ou équivalent sont utilisés dans le présent document dans un sens large et signifie de façon générale « inclut, mais sans s'y limiter.

Le terme "ou" est généralement employé dans un sens large comprenant "et/ou" à moins que le contexte indique clairement le contraire.

Le terme « traitement » doit être compris comme l'action ou suite d'actions ayant pour but d'atteindre un ou plusieurs objectifs thérapeutiques durant un temps défini. Ici, un traitement commence à partir du moment où le patient démarre le système (et/ou branche les connexions fluidiques) jusqu'à ce que le patient arrête ce système (et/ou débranche les connexions fluidiques). Le système définit un ensemble de paramètres (vélocité de la pompe, pression, actionnement, température, surveillance des pressions, des volumes de liquide déplacé, lancement et arrêt des phases,...) pour réaliser un traitement. Un traitement est dit normal si l'ensemble des paramètres permet d'atteindre substantiellement les objectifs thérapeutiques préalablement définis. La durée du traitement est qualifiée de normale si cette durée est substantiellement proche de la durée du traitement normal. Autrement dit, le terme « normal » qualifie ici le fonctionnement / le déroulement du traitement.

Le terme « efficacité » doit être compris comme la qualification d'un effet, ici un traitement. Aussi le terme « efficace » peut être défini comme suit : « qui produit l'effet attendu ». Autrement dit, un traitement efficace doit être compris comme un traitement défini par une prescription et qui a produit l'effet escompté (par exemple quantité d'ultrafiltrat obtenu à la fin du traitement). Ainsi, le terme « efficacité » qualifie ici le résultat du traitement. A travers le terme « efficacité », une notion relative se dégage. En effet, un traitement peut être plus ou moins efficace. Cette efficacité peut sensiblement varié d'un traitement à l'autre et est fonction de nombreuses variables. Dans le présent document, l'efficacité est comparée entre le traitement normal et le traitement effectivement réalisé.

L'expression « Mode de fonctionnement sécurisé » doit être comprise comme un mode opératoire du système qui ne permet pas nécessairement d'atteindre les objectifs prédéfinis ou l'efficacité souhaitée du traitement dit normal. Autrement dit, le mode de fonctionnement dit normal doit être en principe plus efficace qu'un mode de fonctionnement sécurisé. Dans le domaine médical, ce mode de fonctionnement sécurisé doit également répondre à des impératifs de sécurité du patient.

### Concept et méthodes de fonctionnement :

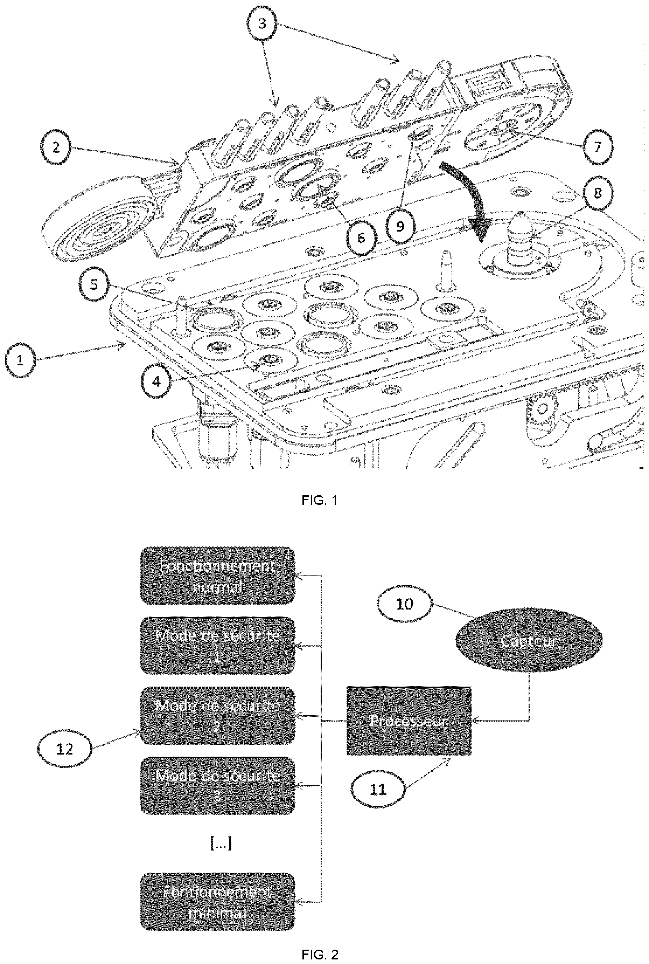

Selon le mode de réalisation de la figure 2, le système comprend un dispositif de contrôle, utilisant au moins un élément du système tel qu'un processeur électronique (11) et un capteur (10). Le système est adapté pour déterminer ou sélectionner un ensemble de paramètres (volume injecté, drainé, chauffage du fluide, pression, débit de la pompe, durée, nombre de cycle et de phase,...) qui peuvent être prédéfinis par le personnelle soignant. Grâce à ce dispositif de contrôle, le système est adapté pour définir ou sélectionner au moins des modes suivants : un mode de fonctionnement normal et un mode de fonctionnement sécurisé. Un mode de fonctionnement sécurisé peut être un mode de fonctionnement minimal. Il peut également exister plusieurs modes de fonctionnement sécurisé intermédiaires. Ces modes sont caractérisés par leur moindre efficacité par rapport au mode normal mais supérieure à celle du mode sécurisé minimal.

Une mémoire connecté au processeur peut être utilisée pour enregistrer les différents modes de fonctionnement et le système est adapté grâce au dispositif de control pour sélectionner un de ces modes de fonctionnement. Un médecin peut pré paramétrer un ou plusieurs différents modes de fonctionnement en fonction de différent scénarios possible (capteur défaillant,...). Un arbre de décision peut être utilisé par le dispositif de contrôle pour choisir le bon mode de fonctionnement. La sélection peut également se faire en cascade où le dispositif de contrôle passe d'un mode de fonctionnement à un autre jusqu'à obtenir un mode de fonctionnement compatible avec les conditions connus par le système.

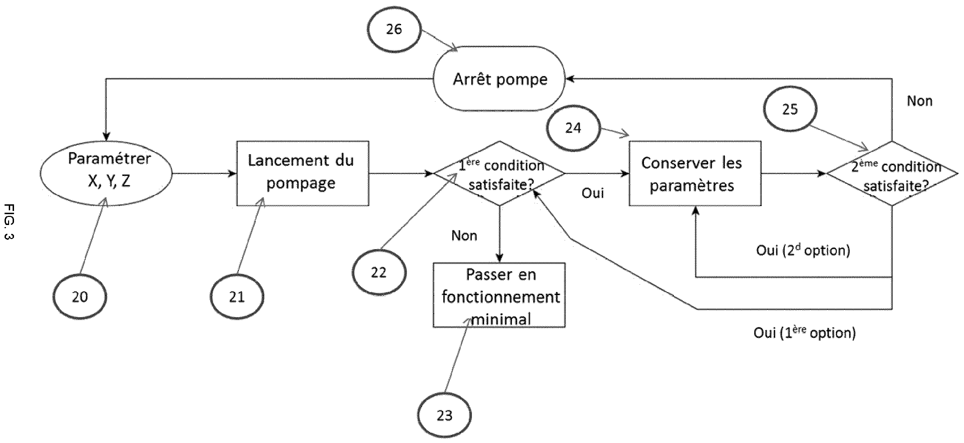

Dans un mode de réalisation, le système est adapté pour fonctionner comme divulgué en figure 3. En début de traitement, le système définit des paramètres (20) en fonction de la prescription définie ou programmée ou donnée par le personnel soignant. Ce premier mode de fonctionnement sera appelé normal. Le système lance la pompe et grâce à des capteurs, le système vérifie ou surveille un ensemble de données. Si un premier ensemble de condition (22) n'est pas respecté alors le système peut commuter dans un mode de fonctionnent sécurisé (qui peut être le mode minimal) permettant de garantir la sécurité du patient et de continuer le traitement alors qu'une condition n'est pas respectée (par exemple un capteur défaillant). Le traitement sera alors sans doute moins efficace, restera sûr, mais le patient aura tout de même reçu un traitement. Il s'agit d'un mode de fonctionnement sécurisé. Si la première condition est respecté alors le traitement peut continuer (ou commencer) avec les paramètres définis précédemment. Un ensemble de conditions peut comprendre une ou plusieurs conditions (non-dépassement d'un seuil et/ou plage de fonctionnement et/ou plage de mesures et/ou moyenne de données et/ou étape correctement achevée,...). Les différents modes de fonctionnement peuvent être caractérisés par un ensemble de paramètres prédéfinis et le système passe d'un mode de fonctionnement à un autre dès qu'une ou plusieurs conditions de fonctionnement n'est pas respecté (dépassement d'un seuil, quantité prescrite de fluide non totalement utilisé, capteur défaillant, données de capteur non cohérent, trop grand écart de mesure entre les différents capteurs,...).

Le système peut être adapté pour surveiller ce premier ensemble de conditions dès le début du traitement et/ou en cours de traitement (périodiquement ou non). Par exemple à chaque début de phase et/ou à intervalles de temps réguliers ou aléatoires. Un deuxième ensemble de conditions peut être vérifié dès le début du traitement et/ou en cours de traitement (périodiquement ou non). Si ce deuxième ensemble de conditions (24) est respecté alors le système peut être adapté pour :
- Revérifier périodiquement le premier ensemble de conditions (1^{ère} option), et/ou
- Garder le mode de fonctionnement précédemment défini (2^{ème} option)
Le système peut effectuer séquentiellement ou en parallèle la vérification des différentes conditions. Ces vérifications peuvent être effectuées une seule fois ou tout au long du traitement à intervalles de temps réguliers ou variables.
Dans le cas où la deuxième condition n'est pas respectée, le système peut décider :
- d'arrêter le traitement ou
- de redéfinir (20) un nouvel ensemble de paramètres afin de continuer le traitement dans un mode de fonctionnement sécurisé mais qui restera moins efficace (par exemple plus long par ce que le nouveau paramétrage définit un débit plus lent) que le mode de fonctionnement normal mais plus efficace que le mode de fonctionnement minimal. Avant de redéfinir ce nouvel ensemble de paramètres, le système peut temporairement arrêter la pompe.

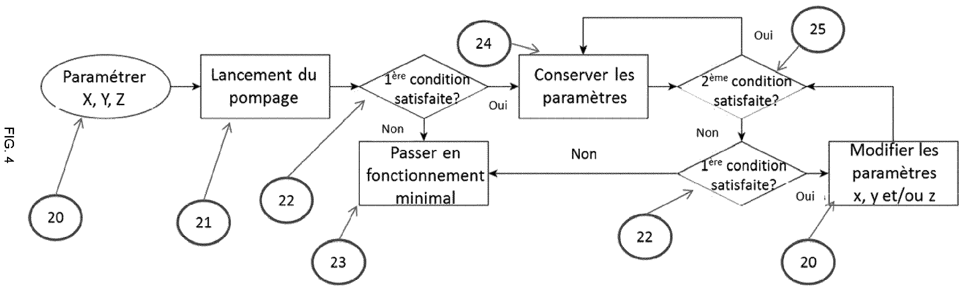

Dans un mode de réalisation, le système est adapté pour fonctionner comme divulgué en figure 4. Le système paramètre en début de traitement un mode de fonctionnement normal et le processus s'effectue dans la majeur partie comme dans la figure 3. Tant que le deuxième ensemble de conditions sera respecté, le système continuera de fonctionner selon le mode de fonctionnement normal. Si le deuxième ensemble de conditions n'est pas respecté, le système vérifiera également le premier ensemble de conditions. Si, le premier ensemble de conditions n'est pas respectée alors le système commutera dans un mode de fonctionnement minimal. Si le premier ensemble de conditions est toujours respectée alors le système modifiera un ou plusieurs paramètres et commutera dans un mode de fonctionnement sécurisé de sorte que le deuxième ensemble de conditions soit respectée. Le deuxième ensemble de conditions peut être adapté en fonction de paramètres prédéfinis. C'est-à-dire que le deuxième ensemble de conditions peut être toujours identique même en cas de changement de mode de fonctionnement ou il peut être modifié. Dans ce dernier cas, une ou plusieurs conditions peuvent être moins contraignantes (par exemple : plage élargir, seuil repoussé, acceptation d'une étape non correctement achevée,...). Tant que le deuxième ensemble de conditions est respectée le système peut garder les paramètres définis précédemment (que ce soit le mode normal ou sécurisé). Le système peut également être adapté pour revenir à un mode de fonctionnement normal au bout d'un certain temps ou en fonction de certaines conditions. Le système vérifiera en boucle les conditions et ajustera au mieux le mode de fonctionnement, en fonction de données qu'il reçoit.

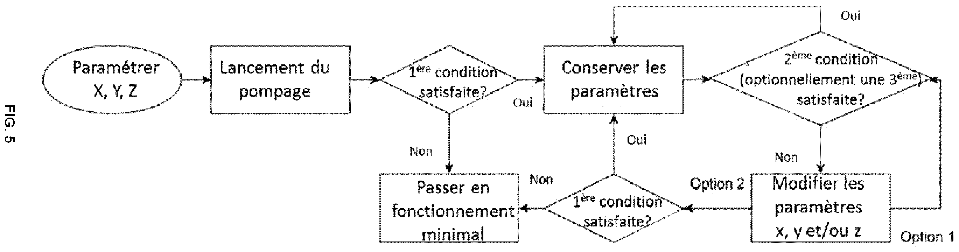

Dans un mode de réalisation, le système est adapté pour fonctionner comme divulgué en figure 5. Le système paramètre en début de traitement un mode de fonctionnement normal et le processus s'effectue en partie comme dans la figure 3 et 4. Le système vérifie un deuxième ensemble de conditions et optionnellement un troisième ensemble de conditions (en même temps ou séquentiellement ou en cas de changement de mode de fonctionnement).

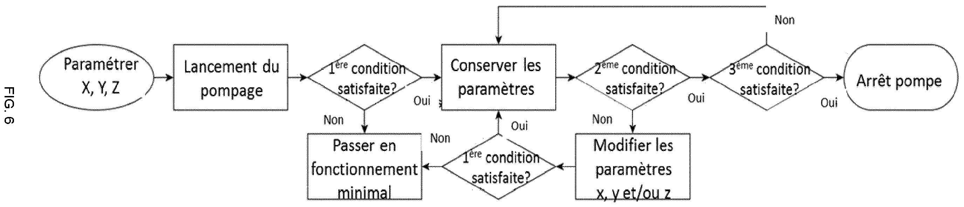

Dans un mode de réalisation, le système est adapté pour fonctionner comme divulgué en figure 6. Le système paramètre en début de traitement un mode de fonctionnement normal et le processus s'effectue en partie comme dans la figure 3, 4 et 5. Dans ce système, le troisième ensemble de conditions est surveillé, dans la mesure ou le deuxième ensemble de condition est respecté. Mais tant que le troisième ensemble de conditions n'est pas respecté le traitement continue selon le mode de fonctionnement précédent. Et lorsque le troisième ensemble de conditions est respecté, alors le traitement s'arrête. Ici la troisième condition peut être le volume injecté, le nombre de cycle programmé, la durée du traitement.

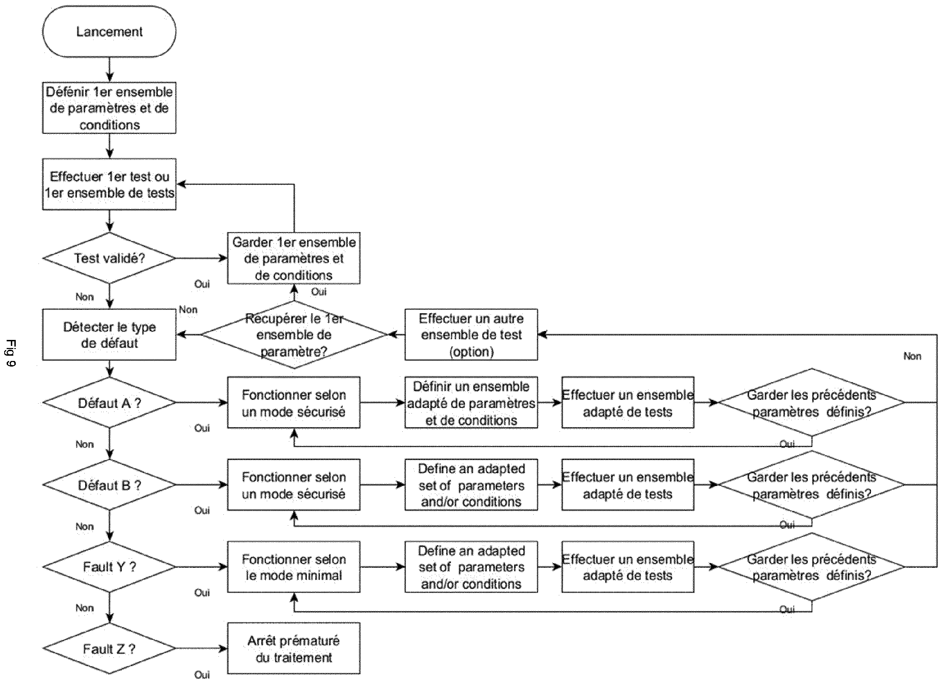

Dans un mode de réalisation divulgué à travers la figure 9, le système comprend un certain nombre de mode sécurisés prédéfinis dont un mode minimal. Par prédéfini, on peut comprendre que le système a déjà un certain nombre de mode sécurisés dont un mode minimal où les paramètres sont tous définis au moins avant le début du traitement. Une stratégie peut être définie pour déterminer (à l'aide d'algorithmes, d'une approche par logique floue, ou d'une approche de type intelligence artificielle par exemple) en fonctions des paramètres du traitement normal et des circonstances rencontrées les nouveaux paramètres du traitement sécurisé. Ces différents paramètres tendront vers la série de paramètres définissant le mode de sécurité minimal. Au cours du traitement, le système procède régulièrement à différents tests et observe le comportement des différents éléments qui le compose. Ces tests peuvent être effectués pendant le traitement ou peuvent nécessiter un arrêt temporaire du traitement. Préférentiellement, ces tests sont effectués par le processeur du système et utilisent des données relatives au fonctionnement du système (pression, température, débit, état des composants,...) et/ou du déroulement du traitement (début/fin de cycle, de phase, quantité restante de dialysat frais, quantité retiré, UF,...). Si au cours de l'un de ces tests ou observation, le système constate ou soupçonne une défaillance ou une condition non rempli, il peut décider de se mettre en mode de fonctionnement sécurisé. Le mode de fonctionnement sécurisé choisi dépendra de l'analyse faite par le système de la défaillance réelle ou supposée. Une fois dans ce mode sécurisé, le système poursuivra le traitement ainsi que les tests et contrôles. Il se peut que le passage dans un premier mode sécurisé rende ces tests et contrôles normaux. Il est aussi possible que ces tests et contrôles restent anormaux mais que dans un tel mode sécurisé la suite du traitement soit sûre pour le patient. Si au cours du temps, les résultats des tests et contrôles redeviennent mauvais ou se dégradent de façon trop prononcée, le système pourra décider de passer dans un deuxième mode de fonctionnement sécurisé, moins efficace que le précédents, mais à nouveau sûr pour le patient. Ce processus peut se répéter plusieurs fois jusqu'à atteindre le mode de sécurité minimal.

Dans un mode de réalisation, le système peut suivre une stratégie où chaque mode de fonctionnement est testé jusqu'à obtenir des résultats de test satisfaisants (Normal → A → B → C →...→ Z). Dans un autre mode de réalisation, un mode de fonctionnement sécurisé pourra être privilégié en fonction des résultats du ou des précédents tests (Normal →A→ D → B). Bien que dans ces exemples, il est fait mention de plusieurs modes de fonctionnement sécurisés qui se succèdent, le système peut simplement passer d'un mode de fonctionnement normal à un mode de fonctionnement sécurisé adapté (Normal → C). Le système peut également être adapté pour revenir à un mode de fonctionnement normal (B → Normal).

A chaque instant, le système peut décider d'arrêter la thérapie s'il considère que celle-ci est suffisamment avancée (selon une série de critères définis à l'avance) ou s'il considère que même le mode de sécurité minimal ne peut garantir la sécurité du patient.

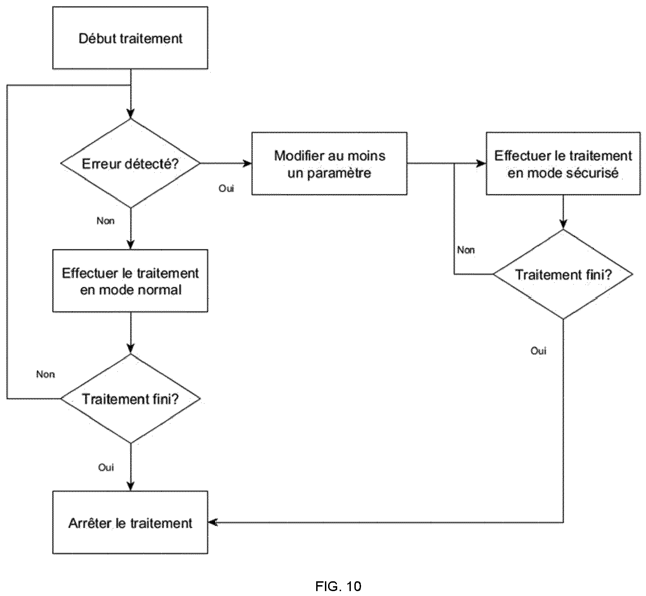

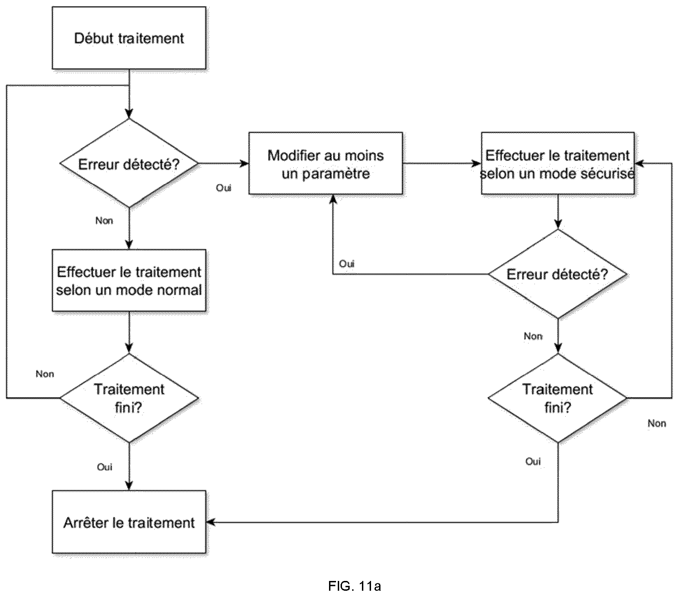

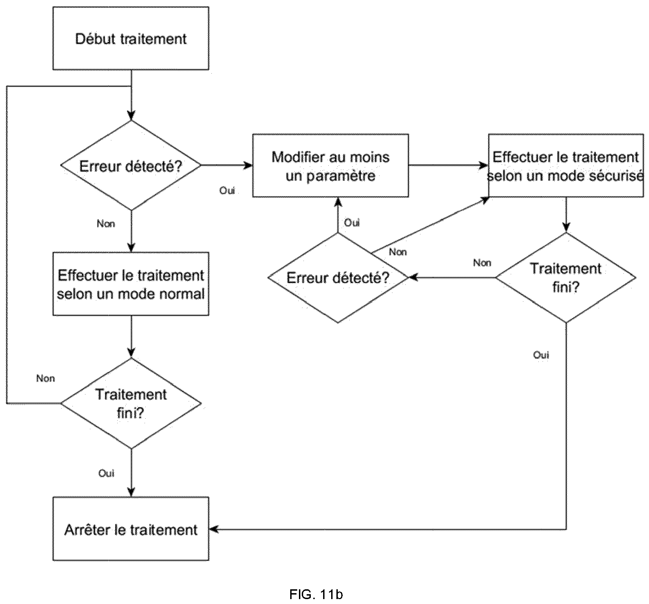

Dans un mode de réalisation, le système est adapté pour fonctionner comme divulgué en figure 10, 11a et 11b, reprenant les concepts présenté ci avant.

### Modes de réalisation et exemples d'utilisation :

Pour une meilleur compréhension du fonctionnement, la description prend l'exemple d'un système de dialyse tel que divulgué dans la figure 1. Le système de dialyse comprend un cycler (1) (ici représenté sans son boitier), une cassette (2). La casette est un élément jetable alors que le cycler est utilisé plusieurs fois avec différentes cassettes. La cassette (2) comprend un mécanisme de pompage (7) qui peut être une pompe péristaltique ou autre (pneumatique,...) des entrées et sorties de fluide (3), des valves (9), des zones d'accouplement avec un capteur (5) du cycler (1). Les entrées et sorties (3) peuvent être adaptées à être connectées via un tube (non représenté) à : un réservoir de dialysat (non représenté), un patient (non représenté), un système de chauffage (non représenté) et/ou un système de remplissage et/ou drainage (non représenté). Le cycler (1) comprend un processeur (non représenté sur le figure 1), des capteurs (5), des actionneurs (4, 8) adaptés pour collaborer avec les valves (9), le mécanisme de pompage (7) de la cassette. La cassette et le cycler sont adaptés pour un accouplement parfait des capteurs et actionneurs avec les éléments de la cassette. Le cycler peut également contenir d'autres capteurs tels que des capteurs de température pour mesurer la température du fluide ou la température ambiante,... Si, par exemple, un capteur est défaillant ou que la cassette est défaillante ou qu'un élément est présent entre le capteur et la cassette ou entre le cycler et la cassette, il se peut que l'accouplement capteur/cassette ne soit pas parfait engendrant une dérive des données ou engendrant des données totalement aberrantes. La membrane recouvrant la zone flexible (6) peut également être défaillante (aspect de surface non conforme, déformation,...).

Comme dans tout système de délivrance de fluide, le volume de fluide délivré ou drainé est une donnée essentielle qu'il faut maitriser. Dans l'état de l'art, il est notamment fait mention de la dangerosité du sur-remplissage de dialysat dans le péritoine du patient. Il est ainsi essentiel de maitriser cette donnée, résultant du volume délivré et/ou drainé. Selon le type de dispositif, il peut être crucial de contrôler la valeur absolue du volume du fluide délivré ainsi que du volume drainé, ou simplement de s'assurer d'un bon contrôle de la balance, c'est-à-dire bien contrôlé la différence entre le volume délivré et le volume drainé. L'estimation de ces volumes peut être effectuée grâce à la pompe elle-même (pompe à piston, pompe péristaltique, ...) et/ou grâce à des capteurs agencés ou non sur la ligne fluidique. Or cette estimation peut être dépendante d'un certain nombre de causes physiques tel que l'usure du système de pompage, la pression en entrée et/ou en sortie de pompe, la température, le cheminement fluidique programmé etc... Ce qui rend difficile l'estimation précise de ces volumes.

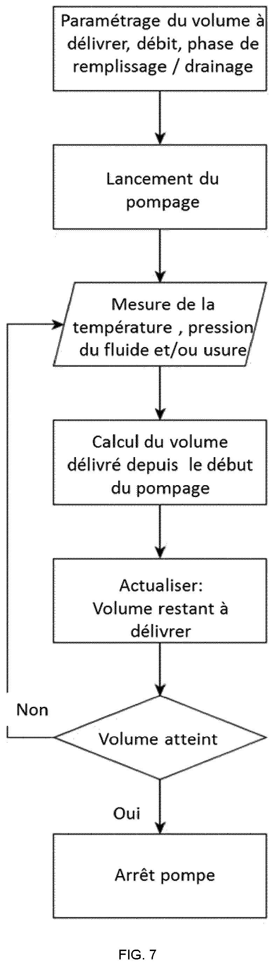

Lors d'une étude préalable, les effets d'au moins un de ces paramètres sur le volume pompé est établi par une théorie physique et/ou une modélisation numérique et/ou par des tests de caractérisation. Ces tests de caractérisation peuvent être effectués suivant un plan de test optimisé afin de réduire le nombre tests nécessaires tout en couvrant la plage nécessaire avec une précision suffisante. De tels plans peuvent être construits sur la base de technique de « Design of Experiment » en utilisant des méthodes connues (méthode Taguchi par exemple) incluant ou non les interactions de ces causes. Les valeurs physiques (par exemple la pression du fluide en entrée de la pompe) quant à elles sont mesurées dans le dispositif par des capteurs. Par la mesure de ces valeurs physiques et avec les effets correspondants préalablement établis, le volume délivré par le système de pompage peut être corrigé pour améliorer sa précision comme divulgué dans la figure 7.

De manière générale, chaque dispositif comprend un nombre déterminé de capteurs (souvent pour des raisons de coût et d'entretien). Le système doit fonctionner avec ce nombre limité de capteurs ce qui contraint le système à fonctionner avec une connaissance non parfaite de l'environnement et de certains facteurs. Par exemple, la position relative du patient et du cycler est une information importante qui aura un impact sur les pressions du fluide déplacé dans la cassette. Théoriquement, le patient ne devrait pas bouger pendant le traitement et les dispositifs ne sont pas dotés de capteur suffisamment précis pour déterminer si le patient bouge ou non pendant le traitement. Or, si le patient change de position, par exemple s'il s'élève de 20 cm par rapport au cycler, cela aura un impact important sur les mesures de la pression du fluide et, potentiellement, aussi sur l'estimation des volumes déplacés. Autrement dit, si le patient bouge le cycler peut détecter qu'une variation de pression a eu lieu, mais il n'en connait pas nécessairement la cause (l'origine d'un tel changement de pression peut effectivement avoir d'autre causes comme par exemple l'apparition d'une restriction dans le chemin fluidique. Le cycler voit au mieux ce changement mais n'as aucun moyen de discerner son origine.). Ainsi, le cycler doit fonctionner au mieux en fonction des circonstances données, en fonction du niveau de connaissance de l'état du système et/ou de l'environnement du patient. Ainsi le système peut-il avoir du mal à juger si la modification de la mesure observée résulte d'un défaut lié au capteur ou d'un mouvement du patient.

Afin de sécuriser les mesures, il est courant de disposer d'au moins une redondance de capteurs (par exemple deux capteurs indépendants sont utilisés pour mesure la pression à l'entrée du dispositif de pompage). Ces deux capteurs sont régulièrement comparés afin de détecter une éventuelle erreur sur l'un des capteurs, provenant par exemple d'une dérive de la mesure ou d'une altération de l'interface entre le capteur et l'environnement à mesurer. Selon l'état de l'art, dès que l'un des capteurs est jugé défaillant, le système entre en alarme et le traitement est interrompu. Le but de l'invention, dans une telle hypothèse, est de continuer le traitement dans un mode dit sécurisé.

Pour plus de clarté, le document présente un système dont les moyens pour calculer les volumes comprennent un capteur de pression. Toutefois, ces moyens peuvent être d'autres éléments comme une chambre volumique ou un pousse seringue utilisé pour la mesure des volumes.

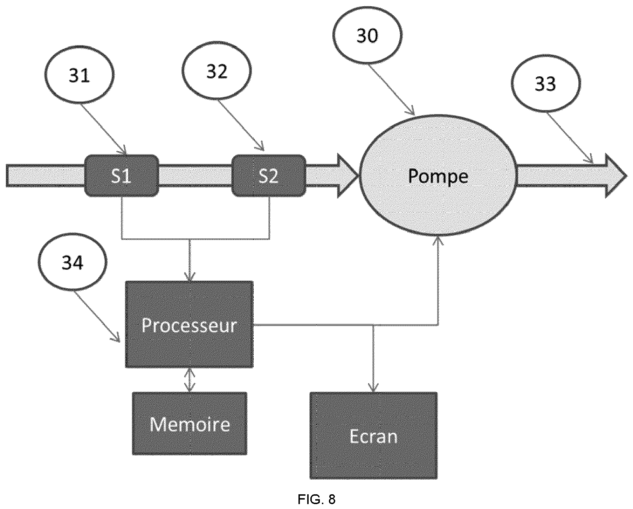

Selon un mode de réalisation présenté en figure 8, le dispositif de dialyse péritonéale dispose de deux capteurs de pression (31, 32) destinés à mesurer la pression à l'entrée d'un système de pompage (30) (par exemple une pompe péristaltique). En fonction de la pression mesurée, le système adapte le débit du système de pompage (30) pour tenir compte des variations de débit en fonction de la dite pression d'entrée. Le processeur (34) analyse les données mesurées par les capteurs de pression (31, 32), estime en fonction d'au moins ces données la quantité de fluide déplacée par la pompe. Le processeur est adapté pour ajuster le fonctionnement de la pompe (vélocité, vitesse de rotation dans le cas d'une pompe péristaltique, débit, durée de fonctionnement,...) en conséquence afin de maintenir un débit effectif correspondant à la prescription.

En cas de défaillance suspectée de l'un des capteurs de pression, le système se met en mode sécurisé. Ce mode de fonctionnement peut réduire le volume d'au moins une phase de fill afin de limiter le remplissage de la cavité péritonéale d'un pourcentage correspondant, par exemple, à la déviation positive maximale possible de pompage ou à une déviation maximale tolérée au-delà de laquelle un risque pourrait résulter pour le patient. Grâce à cette correction, le système s'assure de ne pas sur-remplir le péritoine (un tel sur-remplissage représentant, par exemple, un risque cardio-vasculaire pour le patient), même dans l'hypothèse où le capteur resté fonctionnel venait à être défaillant.

A titre d'exemple, en cas de défaillance de l'un des deux capteurs (ou dans l'hypothèse où l'un des deux capteurs serait potentiellement défaillant), chaque remplissage de la cavité péritonéale selon le cycle suivant sera réduit de 3% du volume programmé. Ce pourcentage peut être prédéfini en fonction du patient et/ou en fonction du design du système (capacité de la pompe,...). Ce pourcentage représente, par exemple, le risque de sur-remplissage lié à cette défaillance ou le sur-remplissage excessif maximal pouvant entrainer un risque pour le patient. Dans cet exemple, il peut s'agir d'une défaillance supposée du fait que l'écart de mesure entre les deux capteurs dépasse un certain seuil, laissant présumer qu'au moins un des deux capteurs est défectueux ou mal couplé avec la zone de mesure (par exemple la membrane de la cassette).

Lors de multiples remplissages, ce pourcentage peut être adapté afin de tenir compte des cumuls de sur-remplissage à chaque cycle (par exemple 8 cycles à 3% représentent un risque maximal de 24% de sur-remplissage). Bien sur le pourcentage peut être adapté pour tenir compte également du moindre drainage dû à la même défaillance à chaque cycle. Ce qui peut représenter, par exemple 24% pour 8 cycles de remplissage auxquels s'ajoutent 24% de moindre drainage, soit un total de 48% de sur-remplissage sur 8 cycles ce qui est proche de la limite tolérée).

Ces pourcentages peuvent naturellement être très différents selon les conditions de remplissage et/ou de drainage et le système définira idéalement au mieux les conditions de réduction de remplissage et/ou d'augmentation de drainage (en cas de drainage partiel) afin de limiter tout risque de dépasser les 50% de sur-remplissage (soit 150% du volume péritonéal, généralement considéré comme limite acceptable). Il est communément admis que 160% ne doit pas en aucun cas être dépassé et que 180% représente un risque sérieux pour la santé du patient. Dans le cas où le cumul de différents cycles risque d'amener à dépasser ces limites de sécurité, il peut être souhaitable d'assurer pendant le traitement un cycle de drainage complet alors que dans le mode de fonctionnement normal le drainage de ce cycle n'aurait pas été complet. Ainsi grâce à ce drainage complet, le système s'assure que la cavité péritonéale est drainée en quasi-totalité, et peut par conséquent recommencer à cumuler les erreurs depuis un ventre vide. Ainsi, le système peut effectuer au moins un drainage complet à intervalle de cycles variable ou fixe qui peut dépendre du pourcentage d'erreur possible. Ce nombre peut être fixé, par exemple, à 6 ou 8 cycles consécutifs.

Dans le cas où un capteur détecte une variation anormale de la pression, alors que le système ne peut en connaitre réellement la cause, le processeur peut décider de modifier le mode de fonctionnement afin de s'adapter à cette variation. Par exemple, avant de lancer le traitement, le système définit certains paramètres tels que le volume délivré, le débit et/ou les phases d'échanges. Le système fonctionnera alors selon un mode de fonctionnement normal. Si les pressions mesurées à l'entrée du système de pompage sont comprises dans une gamme de pression acceptable, le mode de fonctionnement normal sera utilisé tout au long du traitement. Cependant, si à un moment du traitement les mesures dérivent soudainement ou progressivement, puis dépassent un certain seuil alors le système peut commuter dans un autre mode de fonctionnement sécurisé afin de s'adapter à ces mesures. Le système définira au moins un nouvel ensemble de paramètres, par exemple une diminution du débit (par ce que les capteurs de pression ont détecté une augmentation de la pression, ce qui pourrait être dû à une élévation relative du patient par rapport au cycler). Ce mode de fonctionnement peut être considéré comme un mode de fonctionnement sécurisé car il sera éventuellement moins efficace que le mode de fonctionnement normal. Ici, le traitement sera plus lent du fait de la baisse de débit. Si la dérive continue et dépasse un autre seuil, alors le système pourra à nouveau redéfinir un ensemble de nouveaux paramètres.

En réalité, le système ne sait pas si le patient a réellement bougé. Cette variation de pression peut être le fait de plusieurs causes. Toutefois, si cette variation est dû au fait que le patient s'est par exemple surélevé de 20 cm, alors il faut baisser le débit afin d'éviter un sur-remplissage de la cavité péritonéale du patient. C'est pour cette raison que le système redéfinit ces paramètres alors que les autres systèmes de l'état de l'art auraient arrêté le fonctionnement du système. A chaque redéfinition de paramètres, le système peut redéfinir également les seuils.

Selon un autre mode de réalisation, le mode sécurisé tient compte d'erreurs possibles de mesure de température du fluide et, par conséquent, d'erreurs possibles de volume de remplissage et/ou de drainage.

Dans le cas où le système est amené à commuter dans un mode de fonctionnement sécurisé lors d'un seul traitement, le système peut estimer que la cause du problème n'était que temporaire. Dans ce cas, le système peut comprendre un écran ou un moyen d'indication (led de couleur, bruit,...) pour informer le patient qu'un problème a été détecté durant le traitement. Une mémoire pourra enregistrer ces données afin que le patient puisse les transmettre à son docteur ou dans le but d'enregistrer les erreurs de la machine. Idéalement, le système informera le patient que son traitement a été modifié en cours de route et qu'un certain pourcentage du traitement attendu aura au moins été atteint (par exemple 80%, ce qui est de nature à quantifier le minimum thérapeutique obtenu et à adapter les prochains traitements des jours suivants, éventuellement en conséquence).

Dans le cas où le problème se reproduirait à plusieurs reprises c'est-à-dire lors de différents traitements, alors le système peut être adapté pour inciter le patient à procéder à une intervention ou à la requérir ou le système peut, de lui-même, demander auprès du centre d'entretien, une intervention. L'écran pourra conseiller au patient d'effectuer certaines manipulations ou l'inviter à contacter le service de maintenance.

Exemple de fonctionnement d'un mode de réalisation permettant plusieurs adaptations, la valeur de pression mesurée à l'entrée de la pompe atteint une limite, soit parce que le patient s'est déplacé (avec un capteur fonctionnel), soit parce que le capteur de pression dérive. Dans ce deuxième cas, le capteur est défectueux et un risque de sur-remplissage est présent. Pour éviter le sur-remplissage, le débit est diminué (ce qui diminue la pression à l'entrée de la pompe et donc le risque de sur-remplissage). Cette adaptation de débit correspond à l'adaptation n°1. Avec ce nouveau débit, une nouvelle limite de sécurité pour la mesure de pression est calculée. Si cette nouvelle limite est atteinte, le débit est diminué à nouveau ce qui correspond à l'adaptation n°2. Le débit peuvent être ainsi diminué successivement n fois (n adaptations) jusqu'à un débit qui ne représente plus de risque (par exemple parce que le risque de sur-remplissage est réduit en dessous de la limite sécuritaire de 120 à 150%). La conséquence de ce changement de débit aura un impact sur le résultat du traitement. En effet, si le traitement doit être fait sur une durée déterminée alors tout ou partie du dernier cycle ne pourra ne pas être effectué. Dans un cas extrême, plusieurs cycles pourraient ne pas être effectués de manière à respecter la durée de traitement prédéfinie. Ainsi, le résultat du traitement sera de moins bonne qualité/moins efficace que le résultat escompté. Ainsi, l'ensemble des objectifs du traitement ne sera pas respecté. Autrement dit, qu'une partie des objectifs sera respectée, ici au moins la durée du traitement. Dans un autre mode de réalisation, c'est la durée de stase qui peut être privilégiée. Aussi, la durée de stase ne sera pas changée car prédéfinie mais c'est la durée totale du traitement qui augmentera. Le prescripteur peut à l'avance déterminer les objectifs qui ne peuvent être modifié ou qui sont à privilégier en cas de problème. Ainsi, il peut prédéfinir les paramètres qui ne peuvent être changés par le processeur lors du passage en mode de fonctionnement sécurisé.

Dans le cas où le système comprend deux capteurs redondants, la ou les méthodes décrites ci-avant sont particulièrement adapté lorsqu'un ou les deux capteurs sont défaillants ou lorsque la cassette est mal installée dans le cycler ou que le ou les capteurs de pression sont mal couplé à la cassette.

Exemple de fonctionnement d'un mode de réalisation permettant un drain complet, une défaillance est détecté qui, dans le pire des cas, représente un risque de sur-dosage du dispositif de pompage de 6%. Ainsi théoriquement, à chaque cycle, 6% de volume est ajouté au volume déjà présent ce qui représente un volume dans le péritoine de 106 % au premiers cycle, 112% au deuxième cycle etc... Dans cet exemple, deux mesures de protection sont possibles. La première est de diminuer le volume injecté sur les phases de remplissage de 3%. La deuxième est d'imposer une phase de drainage complet au bout de 8 cycles. Au bilan, les erreurs cumulées sur 8 cycles représente un volume maximum dans le péritoine de 100% + 8x3%, soit 124 %, ce qui est un volume acceptable.

### Procédés possibles :

Le document divulgue en outre un procédé pour commander un système médical en fonction d'un traitement défini pour atteindre un ensemble d'objectifs, le procédé peut comprendre les étapes suivantes :
- Fournir un système de dialyse qui comprend :
   ∘ un processeur adapté pour commander le système médical selon au moins deux modes de fonctionnement :
      ▪ un mode de fonctionnement normal déterminé par un premier ensemble de paramètres permettant d'atteindre substantiellement l'ensemble des objectifs définis par le traitement
      ▪ un mode de fonctionnement sécurisé déterminé par un second ensemble de paramètres ne permettant pas d'atteindre l'ensemble des objectifs définis par le traitement mais permettant d'effectuer substantiellement le traitement,
   ∘ un capteur adapté pour envoyer des signaux au processeur,
- Déterminer au moins une condition de fonctionnement,
- Recevoir et analyser les signaux du capteur,
- Sélectionner automatiquement le mode fonctionnement normal ou le mode de fonctionnement sécurisé en fonction de l'analyse des signaux et/ou de ladite au moins une condition de fonctionnement,
- Commander le système médical en fonction du mode de fonctionnent sélectionné.

Le système décrit ci-dessus peut être adapté pour fonctionner selon plusieurs modes de fonctionnement sécurisé. Aussi le processeur peut commuter d'un mode de fonctionnement sécurisé vers un autre mode de fonctionnement sécurisé de façon progressive.

Selon un mode de réalisation, le système médical comprend une pompe commandée par le processeur et adapté pour déplacer un fluide médicale. Le système médical peut par exemple être un système de dialyse.

Optionnellement, le procédé peut comprendre l'étape suivante : Adapter au moins une condition de fonctionnement en fonction du mode de fonctionnement sélectionné.

Selon un mode de réalisation, le paramètre peut être : la durée du traitement, un volume de fluide médical déplacé par la pompe, un volume de fluide médical utilisé, la température ou la pression du fluide déplacé ou le débit de la pompe. Si le dispositif médicale comprend une pompe alors le mode de fonctionnement sécurisé peut être caractérisé par un débit de la pompe moins important qu'en mode de fonctionnement normal.

Préférentiellement, le processeur peut commuter en cours de traitement d'un mode de fonctionnement vers un autre mode de fonctionnement défini en fonction de l'analyse des signaux et/ou de ladite au moins une condition de fonctionnement.

Si le traitement est une dialyse péritonéale alors le système de dialyse peut être adapté pour effectuer plusieurs cycles successifs comprenant un phase d'injection où la système injecte le fluide médical dans le péritoine du patient, une phase de stase où le fluide médical reste un temps déterminé dans le péritoine du patient et une phase de drainage où la pompe retire le fluide du péritoine du patient. Dans ce cas, le (ou les) paramètre peut être : la durée de chaque phase de stase, le volume total de fluide injecté dans et/ou retiré du péritoine, le volume de fluide injecté dans le péritoine lors d'une phase d'injection, le volume de fluide retiré du péritoine lors d'une phase de drainage, le nombre de cycles, la durée des phases ou le débit de la pompe en phase d'injection et/ou de drainage. En outre durant un mode de fonctionnement sécurisé, le processeur peut être adapté pour commander un drainage forcé complet du péritoine au moins une fois avant la fin du traitement. Optionnellement, le processeur peut être adapté pour effectuer plusieurs drainages forcés à intervalles définis.

Selon un mode de réalisation, le mode de fonctionnement sécurisé peut être adapté pour diminuer le risque de sur remplir le péritoine du patient lors du traitement. Et le dispositif médicale peut être adapté pour estimer le risque de sur ou sous remplissage du péritoine du patient.

Préférentiellement, une condition de fonctionnement est : l'état du capteur, la dérive de la mesure du capteur, le dépassement d'un seuil ou d'un domaine prédéfini ou l'écart de mesure avec un autre capteur. Le capteur peut être un capteur de pression ou un capteur de température.

Le document divulgue un autre procédé adapté pour commander un appareil de dialyse. Cet autre procédé peut comprendre les étapes suivantes :
- Observer au moins un paramètre relatif à la dialyse
- Déterminer une première plage de valeur acceptable pour ledit paramètre
Préférentiellement, la commutation d'un mode de fonction à un premier mode de fonction sécurisé si les données dudit au moins un paramètre sont en dehors de ladite première plage de valeur acceptable. Le paramètre observé peut être le volume de dialysat déplacé vers et/ou depuis le péritoine d'un patient.

Le procédé peut également comprendre :
- les étapes additionnelles suivantes :
   ∘ Déterminer une deuxième plage de valeur acceptable pour ledit paramètre
   ∘ commuter du premier mode de fonctionnement dégradé à un deuxième mode de fonctionnement sécurisé, si les données dudit au moins un paramètre sont en dehors de ladite deuxième plage de valeur acceptable.
- Et/ou les étapes additionnelles suivantes :
   ∘ Déterminer une nième plage de valeur acceptable pour ledit paramètre
   ∘ commuter du mode de fonctionnement dégradé n-1 à un mode de fonctionnement sécurisé n si les données dudit au moins paramètre sont en dehors de ladite nième plage de valeur acceptable.

### Autres modes de réalisation possibles :

Le document divulgue un système pour usage médical qui peut comprendre une pompe, des moyens pour estimer un volume de fluide déplacé par la pompe, des moyens pour piloter ladite pompe en fonction des objectifs définis pour le traitement ; dans lequel ladite pompe est adaptée pour délivrer un fluide à un patient ou retirer un fluide d'un patient. Les moyens de pilotage peuvent déterminer un mode de fonctionnement de la pompe en fonction des données envoyées par les moyens d'estimation de volume déplacé. En outre, au moins un mode de fonctionnement peut être un mode sécurisé permettant au système de continuer le traitement afin de se rapprocher d'au moins un des objectifs définis pour le traitement dans le cas ou une partie au moins des moyens de pilotage et/ou d'estimation est potentiellement défaillant, tout en limitant les risques pour le patient.

Les moyens de pilotage peuvent changer de mode de fonctionnement sans l'intervention du patient ou du personnel soignant. Le traitement peut correspondre à celui d'une dialyse péritonéale et peut comprendre au moins deux cycles de phases de Fill et de Drain.

Optionnellement, durant un mode sécurisé, les moyens de pilotage peuvent réduire le volume de fluide déplacé durant au moins une phase de Fill et/ou augmentent le volume de fluide déplacé durant un moins une phase de Drain.

Au moins un des objectifs peut être le temps du traitement, la quantité d'ultrafiltrat retirée, le volume de fluide délivré dans le péritoine du patient et/ou le volume de liquide retiré du péritoine du patient, et/ou le temps de dialyse total effectué. Les moyens d'estimation des volumes déplacés peuvent comprendre un ou plusieurs capteurs de pression ou chambres volumiques. Les moyens d'estimation des volumes déplacés peuvent comprendre un ou plusieurs capteurs de température et/ou viscosité, ou des moyens de calibration. Dans ce ou ces cas, les moyens d'estimation peuvent être redondants et que l'un au moins des deux moyens peut être jugé potentiellement défaillant, conduisant ainsi à activer le mode sécurisé. Les moyens d'estimation redondants peuvent dévier l'un par rapport à l'autre d'au minumum une certaine valeur. La phase de remplissage de chaque cycle en mode sécurisé peut être réduite d'au moins 1% du volume prescrit. La phase de drainage de chaque cycle en mode sécurisé peut être augmentée d'au moins 1% du volume prescrit, dans le cas où le drainage prescrit n'est pas un drainage total. Un drainage le plus complet possible peut être imposé lors d'au moins un drain dès lors que le sur-remplissage potentiel de la cavité péritonéale résultant du cumul des différents cycles précédents dépasse un seuil compris entre 120 et 180%.

Selon un mode de réalisation, l'appareil de dialyse automatisé est adapté pour effectuer une dialyse péritonéale à un patient et il peut comprendre une pompe pilotée par un contrôleur et conçue pour déplacer au moins un premier volume définit de dialysat à partir de moyens d'approvisionnement de dialysat vers la cavité péritonéale du patient durant une phase de Fill et pour retirer au moins un premier volume définit de dialysat de la cavité péritonéale du patient durant une phase de Drain. L'appareil peut en outre comprendre un capteur relié au contrôleur conçu pour estimer les volumes de dialysat déplacé durant au moins l'une de ces deux phases.

Préférentiellement, l'appareil comprend au moins deux modes de fonctionnement dont un mode de fonctionnement permettant d'atteindre l'ensemble des objectifs définis et au moins un mode de fonctionnement sécurisé conçu de manière à se rapprocher d'au moins un desdits objectifs définis sans pour autant l'atteindre, tout en assurant la sécurité du patient. Le mode de fonctionnement peut être déterminé par le contrôleur sans intervention du patient ou du personnel soignant par exemple en fonction de l'estimation des volumes déplacés.

Préférentiellement, le mode de fonctionnement sécurisé peut être actionné par le contrôleur dès lors qu'une erreur dans l'estimation des volumes est possible ou détectée. Le mode de fonctionnement sécurisé peut être déterminé par le contrôleur dès lors que les données du capteur dépassent un certain seuil de mesure ou une différence apr rapport à la mesure attendue. Le mode de fonctionnement sécurisé peut être conçu pour diminuer le débit de la pompe ou la durée du traitement ou au moins un volume de dialysat délivré durant au moins une phase de Fill.

Selon un mode réalisation, l'appareil peut comprendre plusieurs modes sécurisés successifs qui permettent de continuer le traitement mais l'éloigne d'au moins un des objectifs définis. le contrôleur peut sélectionner un des modes sécurisé en fonction des mesures d'au moins un des capteurs. Le contrôleur peut modifier progressivement son mode de fonctionnement jusqu'à ce que les mesures de capteur se situent dans une gamme prédéfinie.

Selon un mode de réalisation, le système de dialyse péritonéale peut comprendre une pompe à liquide, des moyens pour piloter ladite pompe. Où la pompe est adaptée pour délivrer ou retirer un liquide dans ou provenant de la cavité péritonéale d'un patient et le système peut être configuré pour fonctionner selon au moins un des deux modes de fonctionnement suivants:
- Un premier mode de fonctionnement dit normal définissant un premier ensemble de paramètres destiné à atteindre une efficacité donnée du traitement
- Un deuxième mode de fonctionnement dit sécurisé définissant un deuxième ensemble de paramètres destiné à atteindre :
   - une efficacité minimum du traitement, et/ou
   - une efficacité inférieure à l'efficacité du premier mode de fonctionnement, tout en assurant la sécurité du patient durant le traitement.

Dans un autre mode de réalisation, le système peut être configuré pour fonctionner selon au moins un des deux modes de fonctionnement suivants:
- Un premier mode de fonctionnement dit normal respectant les paramètres définis par une prescription donnée
- Un deuxième mode de fonctionnement dit sécurisé ne respectant pas au moins un des paramètres définis par ladite prescription, mais garantissant la sécurité du patient jusqu'à la fin du traitement programmé.

Selon un mode de réalisation possible, l'appareil de dialyse automatisé est adapté pour effectuer une dialyse péritonéale à un patient selon un ensemble de paramètres définis par une prescription donnée garantissant une certaine efficacité du traitement. L'appareil peut comprendre une pompe à liquide, des moyens pour piloter ladite pompe en fonction des paramètres définis par une prescription donnée. Où la pompe est adaptée pour délivrer ou retirer un liquide dans ou provenant de la cavité péritonéale d'un patient et l'appareil est configuré pour modifier au moins un desdits paramètres de manière à effectuer substantiellement l'intégralité du traitement tout en garantissant la sécurité du patient. Toutefois, les nouveaux paramètres peuvent ne pas permettre d'atteindre l'efficacité attendue, en réponse à une déficience au moins partielle suspectée d'un élément (par exemple un capteur, une pompe) d'opérer correctement le dit appareil de dialyse.

## Revendications

1. Système de dialyse adapté pour effectuer un traitement de dialyse à un patient, le système comprenant
- une partie réutilisable comprenant un capteur,
- une partie jetable comprenant un chemin de fluide et une zone de couplage adaptée pour être couplée avec le capteur,
- une pompe adaptée pour déplacer un volume donné de fluide à travers le chemin de fluide, et
- un processeur adapté pour commander la pompe et recevoir des signaux du capteur,
dans lequel le système de dialyse est configuré pour fonctionner selon au moins deux modes de fonctionnement :
- un mode de fonctionnement normal **caractérisé par** un premier débit de la pompe, et
- un mode de fonctionnement sécurisé **caractérisé par** un second débit de la pompe,
dans lequel le processeur est configuré pour :
- commander la pompe selon le mode de fonctionnement normal,
- recevoir les signaux du capteur,
- détecter une anomalie, basé sur les signaux de mesures, et
- commuter automatiquement du mode fonctionnement normal au mode de fonctionnement sécurisé de manière à limiter une erreur d'estimation du volume de fluide déplacé par la pompe tout en continuant le traitement.

2. Système selon la revendication 1, dans lequel le processeur commute d'un mode de fonctionnement sécurisé vers un autre mode de fonctionnement sécurisé de façon progressive.

3. Système selon l'une des précédentes revendications, dans lequel lorsque le système commute en mode de fonctionnement sécurisé, le processeur est configuré pour adapter au moins un des paramètres suivants : la durée du traitement, le volume de fluide déplacé par la pompe, la température ou la pression du fluide déplacé.

4. Système selon l'une des précédentes revendications, dans lequel le mode de fonctionnement sécurisé est **caractérisé par** un débit de la pompe moins important qu'en mode de fonctionnement normal.

5. Système selon l'une des précédentes revendications, dans lequel le processeur commute en cours de traitement d'un mode de fonctionnement vers un autre mode de fonctionnement défini en fonction de l'analyse des signaux.

6. Système selon l'une des précédentes revendications, dans lequel l'anomalie détectée est due à : l'état du capteur, un mauvais couplage entre la zone de couplage et le capteur, la dérive de la mesure du capteur, le déplacement du patient, le dépassement d'un seuil ou d'un domaine prédéfini ou l'écart de mesure avec un autre capteur.

7. Système selon l'une des précédentes revendications, dans lequel le capteur est un capteur de pression ou un capteur de température.

8. Système selon l'une des précédentes revendications comprenant un deuxième capteur redondant.

9. Système selon l'une des précédentes revendications, dans lequel le mode de fonctionnement sécurisé est adapté pour réduire le volume de fluide déplacé par rapport au mode de fonctionnement normal.

10. Système selon l'une des précédentes revendications comprenant un moyen pour informer le patient ou un utilisateur que le système a fonctionné ou fonctionne en mode sécurisé.

11. Système selon l'une des précédentes revendications, dans lequel le chemin de fluide est fluidiquement connecté au péritoine du patient.

12. Système la revendication 11, dans lequel le mode de fonctionnement sécurisé diminue le risque de sur-remplir le péritoine du patient lors du traitement.

13. Système selon l'une des précédentes revendications 11 à 12, dans lequel le processeur est adapté pour estimer le risque de sur- ou sous-remplissage du péritoine du patient.

14. Système selon l'une des précédentes revendications 11 à 13, dans lequel durant un mode de fonctionnement sécurisé, le processeur est adapté pour commander un drainage forcé complet du péritoine au moins une fois avant la fin du traitement.

15. Système selon l'une des précédentes revendications 11 à 14, dans lequel le processeur est adapté pour effectuer plusieurs drainages forcés à intervalles définis.
